# EUROPÄISCHE PATENTSCHRIFT

(11) **EP 1 044 950 B1**
(45) Veröffentlichungstag und Bekanntmachung des Hinweises auf die Patenterteilung: **16.04.2003**
(21) Anmeldenummer: 00101148.5
(22) Anmeldetag: 21.01.2000
(51) Int. Cl.: C07C 17/02, C07C 17/383, C07C 19/045

(54) **Verfahren und Anlage zur Herstellung von 1,2-Dichlorethan**
Method and device for the preparation of 1,2-dichloroethane
Procédé et installation pour la préparation de 1,2-dichloroéthane

(30) Priorität: 14.04.1999 DE 19916753
(43) Veröffentlichungstag der Anmeldung: 18.10.2000
(73) Patentinhaber: Uhde GmbH, 44141 Dortmund (DE)
(72) Erfinder: Porscha, Peter, Dipl.-Ing., 65779 Kelkheim (DE)
(74) Vertreter: Patentanwälte Meinke, Dabringhaus und Partner

(56) Entgegenhaltungen:
- EP-A- 0 075 742
- DE-A- 19 641 562
- US-A- 4 873 384

## Beschreibung

Die Erfindung betrifft ein Verfahren zur Herstellung von 1,2-Dichlorethan (EDC) durch Reaktion von Ethylen und Chlor in der Flüssigphase (Direktchlorierung), wobei aus dem produzierten 1,2-Dichlorethan in einer Hochsiederkolonne und anschließend in einer Vakuumkolonne Hochsieder abgetrennt werden, wobei zur Wärmerückgewinnung und zur Sumpfbeheizung der Kolonnen 1,2-Dichlorethan aus der Direktchlorierung jeweils in einen Wärmeaustauscher zum indirekten Wärmeaustausch mit dem jeweiligen Sumpfprodukt geleitet wird, sowie eine Anlage zur Durchführung eines solchen Verfahrens mit einem Direktchlorierungsreaktor, einer diesem nachgeordneten Hochsiederkolonne und einer der Hochsiederkolonne nachgeordneten Vakuumkolonne, wobei zur Sumpfbeheizung der Kolonnen jeweils ein Wärmeaustauscher vorgesehen ist, dem als Heizmedium 1,2-Dichlorethan aus dem Direktchlorierungsreaktor zugeführt wird.

Die Gewinnung von Vinylchlorid (VCM) aus 1,2-Dichlorethan (EDC) in der Gasphase erfolgt durch Abspaltung von Chlorwasserstoff (HCL). Dabei ist bei diesem Gasphaseverfahren prinzipiell eine katalytische oder thermische Abspaltung von Chlorwasserstoff möglich. Die praktische Anwendung der katalytischen Abspaltung von Chlorwasserstoff scheiterte jedoch bisher an der zu kurzen Lebensdauer der Katalysatoren.

Deshalb hat sich die thermische Abspaltung durchgesetzt, bei der reines und trockenes 1,2-Dichlorethan in einem Pyrolyseofen bei ca. 500°C zu Vinylchlorid und Chlorwasserstoff gespalten wird. Die üblichen Spaltungsraten des 1,2-Dichlorethan liegen dabei zwischen 50 bis 65 %. Das nickt umgesetzte 1,2-Dichlorethan aus der Pyrolyse enthält Verunreinigungen, die bei der endothermen Abspaltung von Chlorwasserstoff entstehen. Der abgespaltene Chlorwasserstoff wird dann in einem weiteren Prozeßschritt, der sogenannten Oxihydrochlorierung, mit Ethylen und Sauerstoff zu 1,2-Dichlorethan umgesetzt.

Das übrige für die Pyrolyse benötigte 1,2-Dichlorethan wird nach dem sogenannten Direktchlorierungsverfahren durch Umsetzung von Ethylen und Chlor in flüssigem 1,2-Dichlorethan hergestellt. Die Reaktion ist exotherm und es ist eine erhebliche Kühlleistung zur Aufrechterhaltung der Reaktionstemperatur erforderlich.

Die Reinheit des 1,2-Dichlorethan, das dem Pyrolyseofen zugeführt wird, muß sehr hoch sein, um einerseits Nebenreaktionen, die zur Verschmutzung der Pyrolyserohre führen können, zu unterdrücken und andererseits eine hohe Qualität des Vinylchlorids zu erreichen. Deshalb wird das nicht umgesetzte 1,2-Dichlorethan aus dem Pyrolyseofen, das 1,2-Dichlorethan aus der Oxihydrochlorierung und, falls erforderlich, das 1,2-Dichlorethan aus der Direktchlorierung in einer energieaufwendigen 1,2-Dichlorethan-Destillation gereinigt.

Bei diesem Verfahren stellt der Direktchlorierungsreaktor einen der größten Verbraucher für Kühlenergie und die Kolonne zur Abtrennung von Hochsiedern einen der größten Verbraucher für Heizenergie innerhalb einer EDC-/VCM-Anlage dar. Aus wirtschaftlichen Erwägungen sind deshalb bereits Verfahren vorgeschlagen worden, um den Energieverbrauch zu verringern. Zwar wird bei diesen Verfahren die Reaktionswärme der Direktchlorierung zur Beheizung der EDC-Destillation durch direkte oder indirekte Beheizung ausgenutzt bzw. nur Heizenergie eingespart, indem das Prinzip Rektifikation mit Brüdenverdichtung in der EDC-Destillation angewendet wird, aber diese Verfahren sind im wesentlichen mit den folgenden Nachteilen behaftet:

Grundsätzlich ist es beispielsweise aus DE 29 35 884 A1 oder DE 24 27 045 A1 bekannt, die Produktdämpfe des Direktchlorierungsreaktors in den Sumpf der Hochsiederkolonne einzuleiten und somit eine direkte Beheizung der Hochsiederkolonne zu bewirken. Dies führt jedoch dazu, daß der Durchmesser der Hochsiederkolonne und die Fläche des Rücklaufkondensators sehr groß werden müssen, da bei der exothermen Siedereaktion ohne Fremdkühlung je Einheit neu gebildetes 1,2-Dichlorethan in der Direktchlorierung die mehrfache Menge an 1,2-Dichlorethan verdampft wird und zusätzlich mit den anderen 1,2-Dichlorethanströmen in der Hochsiederkolonne rektifiziert wird. Ferner ist von Nachteil, daß, da gleichzeitig vom Sumpf der Hochsiederkolonne flüssiges 1,2-Dichlorethan (ebenfalls die mehrfache Menge des neu gebildeten Dichlorethans im Direktchlorierungsreaktor) zurück zum Reaktor gefahren werden muß, man gezwungenermaßen ein erhöhtes Niveau an höhersiedenden Nebenprodukten im Reaktor hat, welches sich einerseits negativ auf die Katalysatorwirksamkeit auswirkt und zudem andererseits noch die weitere Nebenproduktbildung fördert, was letztendlich zu einer Ausbeuteverschlechterung führt. Aufgrund dessen kann die Reaktionswärme der Direktchlorierung auch nicht für die direkte Beheizung der Vakuumkolonne der EDC-Destillation genutzt werden, da hier die Konzentration an Hochsiedern im Sumpf größer als 90 % ist.

Neben dieser direkten Beheizung sind auch gattungsgemäße indirekte Beheizungsverfahren bekannt, und zwar durch Beheizen der Kolonnen-Reboiler der EDC-Destillation mittels heißem Reaktorkreislauf-1,2-Dichlorethan oder dampfförmigem 1,2-Dichlorethan vom Direktchlorierungsreaktor. Zu diesem Zweck werden bei diesen Verfahren, welche beispielsweise in DE 41 33 810 A1, DE 40 39 960 A1 oder EP 0 075 742 B1 beschrieben sind, als Wärmeaustauscher Naturumlaufverdampfer eingesetzt, was es notwendig macht, bei dem vorhandenen Stoffsystem 1,2-Dichlorethan für eine einwandfreie Beheizung eine treibende Temperaturdifferenz von ca. 20 bis 25°C zu gewährleisten. Dies hat die folgenden nachteiligen Konsequenzen: Man muß die Reaktionstemperatur des Reaktors gegenüber den Sumpftemperaturen der Kolonnen der EDC-Destillation relativ stark erhöhen, um die Reaktionswärme indirekt an die Naturumlaufverdampfer übertragen zu können. Dies führt zu einer Verschlechterung der Chlor- und Ethylenausbeute und zur erhöhten Nebenproduktbildung.

Die Reaktionswärme des Reaktors kann auch bei relativ niedrigen Reaktionstemperaturen an die Reboiler der EDC-Destillation abgeführt werden. Allerdings müssen dann die Destillationskolonnen unter Vakuum gesetzt werden. Dies hat zur Folge, daß vor allem der Durchmesser und der Rücklaufkondensator der Hochsiederkolonne sehr groß werden und ein erhöhter Bedarf an Energie zur Aufrechterhaltung des Vakuums notwendig ist, was sich ebenfalls negativ auf die Wirtschaftlichkeit des Verfahrens auswirkt.

Schließlich sind auch Verfahren der Rektifikation mit Brüdenverdichtung bekannt, dabei werden die Kopfbrüden der Hochsiederkolonne mittels eines Kompressors und Einsatz dieser Brüden als Heizmittel für den Sumpfaufheizer der gleichen Kolonne verdichtet (DE 34 40 685 A1). Der Einsatz eines Kompressors mit rotierenden Teilen ist jedoch somit erforderlich, welcher einen hohen Anschaffungspreis aufweist und Instandhaltungskosten und Ersatzteilkosten verursacht. Dabei werden zwar an der Hochsiederkolonne Dampf- und Kühlwasserkosten eingespart, dafür steigen aber die Kosten für elektrische Energie erheblich an. Außerdem ist von weiterem Nachteil, daß die Integration der Reaktionswärme des Direktchlorierungsreaktors bei diesem Konzept nicht berücksichtigt wird.

Aufgabe der Erfindung ist es deshalb, eine Lösung zu schaffen, bei der die Reaktionsenthalpie der Direktchlorierung in variabler Weise benutzt werden kann, ohne dafür die vorstehend beschriebenen Nachteile in Kauf nehmen zu müssen.

Diese Aufgabe wird bei einem Verfahren der eingangs bezeichneten Art erfindungsgemäß dadurch gelöst, daß zur Sumpfbeheizung der Kolonnen wenigstens ein Fallstromverdampfer eingesetzt wird, dessen oberseitigem Flüssigkeitsverteiler das jeweilige Sumpfprodukt zugeführt wird.

Fallstromverdampfer sind für andere Anwendungszwecke grundsätzlich bekannt und zeichnen sich dadurch aus, daß sie sowohl mit großen als auch mit theoretisch beliebig kleinen Temperaturdifferenzen betrieben werden können. Bei einem solchen Fallstromverdampfer strömen Flüssigkeit und Brüden im Gleichstrom nach unten. Eine entscheidende Rolle für den störungsfreien Betrieb spielt die gleichmäßige und ausreichende Benetzung der Heizflächen mit Flüssigkeit. Aufgrund der Befürchtung, daß die Verschmutzungen im Sumpf der EDC-Kolonnen den empfindlichen Flüssigkeitsverteiler eines Fallstromverdampfers teilweise blockieren und es somit zu einer ungleichmäßigen Flüssigkeitsverteilung der Heizrohre kommt, bestand bisher in der Fachwelt ein Vorurteil gegen den Einsatz von Fallstromverdampfern als Sumpfaufheizer bei der EDC-Destillation. Eine ungleichmäßige Flüssigkeitsverteilung im Fallstromverdampfer führt nämlich zu trockenen Stellen, Verkrustungen und in der Folge zum vollständigen Verstopfen einzelner Rohre.

Überraschenderweise hat sich herausgestellt, daß bei Einsatz eines Fallstromverdampfers, der bei kleinen Temperaturdifferenzen betrieben wird, auch hinsichtlich des Stoffüberganges ein Vorteil erzielt wird. Es hat sich nämlich gezeigt, daß die Trennwirkung bei den geringen Wärmestromdichten dergestalt verbessert wird, daß bei vorgegebenem Wärmestrom ein größerer Anteil der leichter flüchtigen Komponente in die Dampfphase übergeht. Dies ist vor allem für den Betrieb der Vakuumkolonne ein ganz wichtiger Aspekt, da auf diese Weise der Anteil an Produkt (=EDC) im Sumpfabzug der Vakuumkolonne reduziert wird. Der Sumpfabzug besteht zum größten Teil aus Hochsiedern und muß in einer Rückstandsverbrennung entsorgt werden.

Ferner hat sich entgegen des bestehenden Vorurteils überraschend auch herausgestellt, daß der Verschmutzungswiderstand der Heizflächen der Fallstromverdampfer gegenüber den bisher eingesetzten Naturumlaufverdampfern gering ist. Üblicherweise werden industrielle Sumpfverdampfer bei vorgegebener äußerer Bilanz der Trenneinheit meist mit konstantem Wärmestrom betrieben. Verringert sich aufgrund von Verschmutzung der Wärmedurchgangskoeffizient, reagiert das System mit einer Vergrößerung der treibenden Temperaturdifferenz. Der zeitliche Verlauf des Heizdampfdruckes ist also ein Indikator für das Fortschreiten der Belagbildung auf der Wärmeübergangsfläche. Der Vergleich der Verschmutzungswiderstände beim Naturumlaufverdampfer und Fallstromverdampfer hat gezeigt, daß zwar der zeitliche Verlauf qualitativ identisch beschrieben werden kann, jedoch die absoluten Zahlenwerte beim Fallstromverdampfer geringer sind.

Neben diesen erheblichen Vorteilen des erfindungsgemäßen Verfahrens wird durch das erfindungsgemäße Verfahren auch ein Beitrag zum Umweltschutz geleistet, da im Sumpf der Vakuumkolonne mehr 1,2-Dichlorethan zurückgewonnen werden kann und die Wärmeübertragungsflächen der Verdampfer weniger verschmutzen. Ferner kann mit einem Fallstromverdampfer pro Apparateeinheit eine deutlich größere Wärmeübertragungsfläche realisiert werden als mit den bisher eingesetzten Naturumlaufverdampfern. Dies bedeutet, daß bei großer Anlageleistung die Sumpfbeheizung einer EDC-Kolonne mit nur einer einzigen Fallstromverdampfereinheit vorgenommen werden kann, während bei Verwendung eines Naturumlaufverfdampfers mehrere Einheiten notwendig sind. Dies spart Investitionskosten und Grundflächenbedarf. Ferner besitzt ein Fallstromverdampfer gegenüber einem Naturumlaufverdampfer einen geringeren Flüssigkeitsinhalt und weist daher ein sehr spontanes Regelverhalten auf. Dies ist ebenfalls sehr vorteilhaft, um im Sumpfaustrag der Vakuumkolonne geringe Restkonzentrationen an Leichtsieder (=EDC) zu realisieren.

In weiterer vorteilhafter Ausgestaltung ist vorgesehen, daß die Direktchlorierung bei Temperaturen von 75 bis 125°C und Drücken von 0,8 bis 3 bar durchgeführt wird. Der Verdampfungsdruck im Kolonnenverdampfer (Fallstromverdampfer) ist dann nur maximal 50 mbar größer als der Druck im Sumpf der zu beheizenden Kolonne. Ferner ist der Wärmeübergangskoeffizient auf der Verdampfungsseite nahezu unabhängig von der treibenden Temperaturdifferenz (bei gegebenem Sumpfdruck der zu beheizenden Kolonne ist der Unterschied des Wärmeübergangskoeffizienten auf der Verdampfungsseite zwischen einer treibenden Temperaturdifferenz von 3°C und 25°C kleiner als 3 %).

Die Erfindung schlägt auch eine Anlage zur Durchführung des Verfahrens nach dem Oberbegriff des Patentanspruches 3 vor, die sich dadurch auszeichnet, daß die Wärmeaustauscher als Fallstromverdampfer ausgebildet sind.

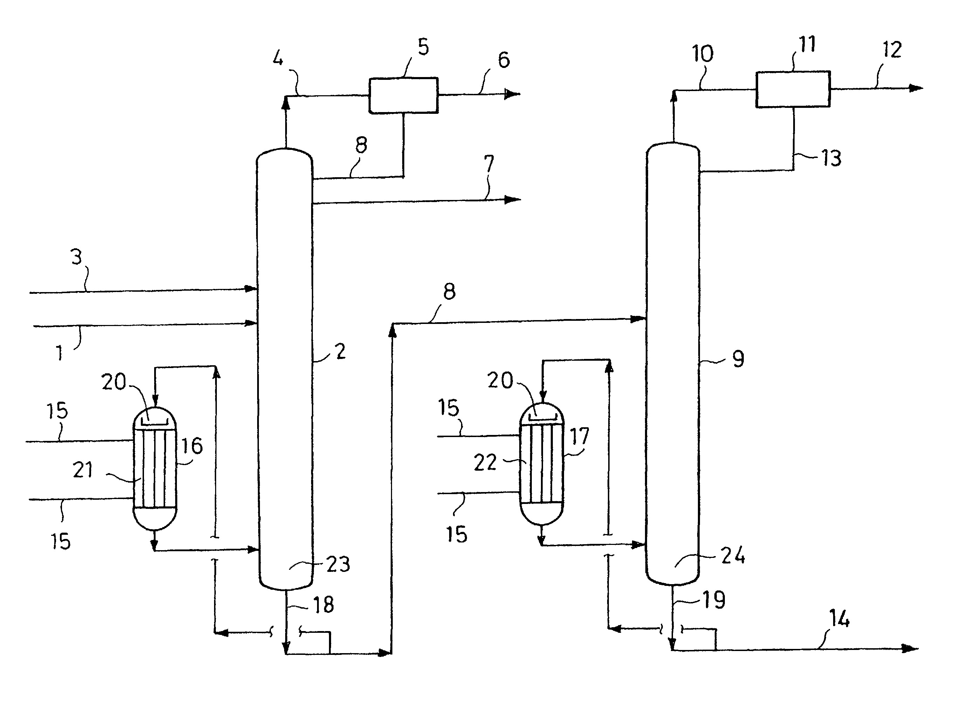

Die Erfindung ist nachstehend anhand der Zeichnung beispielsweise näher erläutert. Diese zeigt in der einzigen Figur ein vereinfachtes Verfahrensfließbild einer Anlage zur Durchführung eines erfindungsgemäßen Verfahrens.

In der Zeichnung sind von der Anlage nur die für die Erfindung wesentlichen Teile dargestellt.

In der EDC-Destillation wird das EDC aus der Oxihydrochlorierung und das nicht umgesetzte EDC aus dem Pyrolyseofen in einer energieaufwendigen EDC-Destillation gereinigt. Falls erforderlich, kann auch das EDC von der Direktchlorierung in der EDC-Destillation gereinigt werden.

Das EDC der Oxihydrochlorierung wird in einer Entwässerungs-/Leichtsiederkolonne, die in der Zeichnung nicht dargestellt ist, zunächst von Wasser und Leichtsiedern getrennt. Anschließend wird das EDC der Oxihydrochlorierung, welches noch Hochsieder enthält, über eine Leitung 1 einer Hochsiederkolonne 2 zugeführt. Nicht umgesetztes EDC aus einem nicht dargestellten Pyrolyseofen enthält ebenfalls Hochsieder und wird über eine Leitung 3 ebenfalls der Hochsiederkolonne 2 zugeführt. In der Hochsiederkolonne 2 werden die zugeführten Stoffströme fraktioniert destilliert.

Gereinigtes EDC wird am Kopf der Hochsiederkolonne 2 über eine Leitung 4 abgezogen, in einem Wärmeaustauscher 5 kondensiert und entweder über eine Leitung 6 oder eine Leitung 7 als reines EDC gewonnen. Der Rückfluß wird der Hochsiederkolonne 2 über eine Leitung 8 zugeführt.

Im Sumpf der Hochsiederkolonne reichem sich die Hochsieder an. Eine verstärkte Reinigung des Sumpfes der Hochsiederkolonne 2 ist möglich, indem der Sumpfstrom über eine Leitung 8 einer Vakuumkolonne 9 zugeführt wird. Gereinigtes EDC wird am Kopf der Vakuumkolonne 9 über eine Leitung 10 abgezogen, in einem Wärmeaustauscher 11 kondensiert und über eine Leitung 12 als reines EDC gewonnen. Der Rückfluß wird der Vakuumkolonne über eine Leitung 13 zugeführt.

Der Sumpfabzug 14 der Vakuumkolonne 9 besteht aus Hochsiedern und einem kleinen Anteil EDC.

Erfindungsgemäß ist die Beheizung der Kolonnen 2, 9 nun folgendermaßen:

Über Leitungen 15 wird entweder ein EDC-Flüssigkeitsstrom oder ein EDC-Dampfstrom von dem nicht dargestellten Direktchlorierungsreaktor einem zur Sumpfbeheizung der Hochsiederkolonne 2 verwendeten Fallstromverdampfer 16 und einem zur Sumpfbeheizung der Vakuumkolonne 9 verwendeten Fallstromverdampfer 17 als Heizmedium zu- und abgeführt. Vom Sumpf der Hochsiederkolonne 18 und vom Sumpf der Vakuumkolonne 19 wird jeweils Flüssigkeit zu einem speziellen Flüssigkeitsverteiler 20 am Kopf der Fallstromverdampfer 16 bzw. 17 gefördert, der so ausgeführt ist, daß es bei den auftretenden Verschmutzungen nicht zu ungleichmäßiger Flüssigkeitsverteilung, zu trockenen Stellen, Verkrustungen und in der Folge zum Verstopfen der Rohre kommen kann.

Vom Kopf des Verdampferkörpers fließt die Flüssigkeit jeweils als gleichmäßig verteilter siedender Film aufgrund der Schwerkraft auf der Innenseite der Heizrohre der Fallstromverdampfer 16, 17 ab und verdampft dabei teilweise. Die effektive treibende Temperaturdifferenz für die Verdampfung, definiert als mittlere Temperatur 21 bzw. 22 auf der Beheizungsseite und der Sumpftemperatur 23 bzw. 24 der Kolonne 2 bzw. 9, kann dabei beliebig klein sein und unterliegt keinen Beschränkungen bezüglich kleiner Temperaturdifferenzen, wie bei Naturumlaufverdampfern.

Die Erfindung wird nachfolgend mit Bezugnahme auf die Zeichnung auch noch anhand spezieller Verfahrensbeispiele erläutert:

### Beispiel 1

Über die Leitung 3 wurden 23.1 to/h (Tonnen pro Stunde) nicht umgesetztes EDC aus einem nicht dargestellten Pyrolyseofen und über die Leitung 1 wurden 16, 2 to/h EDC aus der Oxihydrochlorierung der Hochsiederkolonne 2 zugeführt. Beide Ströme enthielten Hochsieder und mußten franktioniert destilliert werden.

Die Sumpftemperatur der Kolonne 2 betrug dabei 100°C bei einem Druck von 1,5 bar, die Kopftemperatur betrug 87°C bei einem Druck von 1,2 bar. Die Kolonne 2 wurde mit einem Rücklaufverhältnis von R=0,5 betrieben.

Der Fallstromverdampfer 16 wurde zur Beheizung über die Leitungen 15 mit einem EDC-Flüssigkeitsstrom von einem nicht dargestellten Direktchlorierungsreaktor beaufschlagt. Die Kreislaufmenge betrug dabei 870 to/h, die Vorlauftemperatur war 115°C und die Rücklauftemperatur 103°C.

### Beispiel 2

Die Kolonne 2 wurde unter den gleichen Bedingungen wie in Beispiel 1 betrieben, jedoch wurde dieses Mal ein EDC-Dampfstrom von einem nicht dargestellten Direktchlorierungsreaktor zur Beheizung des Fallstromverdampfers 16 verwendet. In der Zuleitung 15 zum Fallstromverdampfer 16 waren die Betriebsbedingungen des kondensierten EDC-Dampfes 108°C und 1,9 bar und es strömten insgesamt 50,5 to/h EDC-Dampf.

### Beispiel 3

Über die Leitung 8 wurden 2,8 to/h hochsiederhaltiges EDC der Vakuumkolonne 9 zugeführt. Die Sumpftemperatur der Kolonne 9 betrug dabei 89°C bei einem Druck von 0,4 bar, die Kopftemperatur betrug 45°C bei einem Druck von 0,26 bar. Die Kolonne 9 wurde dabei mit einem Rücklaufverhältnis von 1,0 betrieben. Der Sumpfstrom 14 der Vakuumkolonne bestand aus 0,3 to/h Hochsieder und enthielt lediglich 3 % EDC. Der Fallstromverdampfer 17 wurde zur Beheizung über die Leitungen 15 mit einem EDC-Dampf aus einem hier nicht dargestellten Entspannungsgefäß eines Direktchlorierungsreaktors beaufschlagt.

In der Zuleitung 15 zum Fallstromverdampfer 17 waren die Betriebsbedingungen des kondensierenden EDC-Dampfes 98°C und 1,6 bar und es strömten insgesamt 8,1 to/h EDC-Dampf.

## Patentansprüche

1. Verfahren zur Herstellung von 1,2-Dichlorethan (EDC) durch Reaktion von Ethylen und Chlor in der Flüssigphase (Direktchlorierung), wobei aus dem produzierten 1,2-Dichlorethan in einer Hochsiederkolonne und anschließend in einer Vakuumkolonne Hochsieder abgetrennt werden, wobei zur Wärmerückgewinnung und zur Sumptbeheizung der Kolonnen 1,2-Dichlorethan aus der Direktchlorierung jeweils in einen Wärmeaustauscher zum indirekten Wärmeaustausch mit dem jeweiligen Sumpfprodukt geleitet wird,
**dadurch gekennzeichnet,**
**daß** zur Sumpfbeheizung der Kolonnen wenigstens ein Fallstromverdampfer eingesetzt wird, dessen oberseitigem Flüssigkeitsverteiler das jeweilige Sumpfprodukt zugeführt wird.

2. Verfahren nach Anspruch 1,
**dadurch gekennzeichnet,**
**daß** die Direktchlorierung bei Temperaturen von 75 bis 125°C und Drücken von 0,8 bis 3 bar durchgeführt wird.

3. Anlage zur Durchführung des Verfahrens nach Anspruch 1 oder 2 mit einem Direktchlorierungsreaktor, einer diesem nachgeordneten Hochsiederkolonne und einer der Hochsiederkolonne nachgeordneten Vakuumkolonne, wobei zur Sumpfbeheizung der Kolonne jeweils ein Wärmeaustauscher vorgesehen ist, dem als Heizmedium 1,2-Dichlorethan aus dem Direktchlorierungsreaktor zugeführt wird,
**dadurch gekennzeichnet,**
**daß** die Wärmeaustauscher als Fallstromverdampfer (16,17) ausgebildet sind.

## Claims

1. Method for the production of 1,2-dichloroethane (EDC) through reaction of ethylene and chlorine in the liquid phase (direct chlorination), wherein from the 1,2-dichloroethane that has been produced, in a high-boiling column and subsequently in a vacuum column high-boiling [heavy constituents] are separated off, wherein for heat recovery and for sump heating of the columns, 1,2-dichloroethane from the direct chlorination is respectively guided into a heat exchanger for indirect heat exchange with the respective sump product,
**characterised in that**
for the sump heating of the columns, at least one downdraft evaporator is used, with the respective sump product being supplied to its liquid distributor on the upper side.

2. Method in accordance with claim 1, **characterised in that** the direct chlorination is carried out at temperatures of 75 to 125°C and pressures of 0.8 to 3 bar.

3. Plant for carrying out the method according to claim 1 or 2, with a direct chlorination reactor, a high-boiling column located downstream of this, and a vacuum column arranged downstream of the high-boiling column, wherein for the sump heating of the column, in each case a heat exchanger is provided, to which 1,2-dichloroethane is supplied as a heating medium from the direct chlorination reactor,
**characterised in that**
the heat exchangers are designed as downdraft evaporators (16, 17).

## Revendications

1. Procédé de production du 1,2-dichloroéthane (DCE) en faisant réagir de l'éthylène et du chlore en phase liquide (chloration directe), dans lequel les fractions lourdes sont séparées du 1,2-dichloroéthane produit dans une colonne pour fractions lourdes, et ensuite dans une colonne sous vide, dans lequel, pour récupérer de la chaleur et pour chauffer le réservoir de la colonne, le 1,2-dichloroéthane issu de la chloration directe est placé à chaque fois dans un échangeur thermique pour un échange indirect de chaleur avec le résidu de distillation correspondant,
**caractérisé en ce que**, pour chauffer le réservoir de la colonne, au moins un rebouilleur à circulation forcée est installé, dont la partie supérieure du répartiteur de liquide reçoit le résidu de distillation correspondant.

2. Procédé selon la revendication 1, **caractérisé en ce que** la chloration directe est réalisée à des températures de 75 à 125°C et à des pressions de 0,8 à 3 bar.

3. Installation pour la réalisation du procédé selon les revendications 1 ou 2 avec un réacteur de chloration directe, une colonne pour fractions lourdes couplée à ce réacteur, et une colonne sous vide couplée à la colonne pour fractions lourdes, installation dans laquelle, pour chauffer le réservoir de la colonne, un échangeur thermique correspondant est prévu, lequel reçoit le 1,2-dichloroéthane, en tant que fluide caloporteur, issu du réacteur de chloration directe,
**caractérisée en ce que** les échangeurs thermiques sont configurés comme rebouilleurs à circulation forcée (16, 17).
